# EUROPÄISCHE PATENTSCHRIFT

(11) **EP 0 271 854 B1**
(45) Veröffentlichungstag und Bekanntmachung des Hinweises auf die Patenterteilung: **31.03.1993**
(21) Anmeldenummer: 87118448.7
(22) Anmeldetag: 12.12.1987
(51) Int. Cl.: G01N 33/52, C12Q 1/28

(54) **Testträger für die analytische Bestimmung von Bestandteilen von Körperflüssigkeiten**
Test strip for the analytical determination of body fluid constituents
Bande-analyse pour la détermination analytique de composants dans les fluides corporels

(30) Priorität: 19.12.1986 DE 3643516
(43) Veröffentlichungstag der Anmeldung: 22.06.1988
(73) Patentinhaber: BOEHRINGER MANNHEIM GMBH, 68298 Mannheim (DE)
(72) Erfinder: Berger, Dieter, Dr. rer. nat., D-6806 Viernheim (DE); Knappe, Wolfgang-Reinhold, Dr. rer. nat., D-6842 Bürstadt (DE); Lorenz, Robert, D-6700 Ludwigshafen (DE); Grage, Henry Martin, Jr., Carmel, IN 46032 (US); Skarstedt, Mark Teofil, Indianapolis, IN 46064 (US); Sojka, Bernward, Dr. rer. nat., D-6806 Viernheim (DE); Bleisteiner, Manfred, D-6800 Mannheim 51 (DE)

(56) Entgegenhaltungen:
- EP-A- 0 113 896
- EP-A- 0 182 373
- EP-A- 0 208 952
- DE-A- 2 655 977
- DE-C- 3 445 816

## Beschreibung

Die Erfindung betrifft einen Testträger für die analytische Bestimmung von Bestandteilen von Körperflüssigkeiten aus einer bestimmten maximalen Probenmenge mit einer Tragschicht und mehreren darauf angeordneten Testschichten, die wenigstens teilweise in Flüssigkeitsaustausch ermöglichendem Kontakt zueinanderstehen, wobei eine Aufgabezone für die Aufgabe einer Probe der Körperflüssigkeit, eine Nachweiszone enthaltend mindestens eine Nachweisschicht für die Erzeugung eines nachweisbaren für die analytische Bestimmung charakterischen Signals und eine Saugzone aus einem saugfähigem Material, in dieser Reihenfolge im wesentlichen nebeneinander auf der Tragschicht angeordnet sind.

Zur qualitativen oder quantitativen analytischen Bestimmung von Bestandteilen von Körperflüssigkeiten, insbesondere von Blut werden in jüngerer Zeit zunehmend sogenannte trägergebundene Tests verwendet. Bei diesen sind Reagenzien in entsprechenden Schichten eines festen Testträgers eingebettet, der mit der Probe in Kontakt gebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann.

Testträger sind häufig als Teststreifen ausgebildet, die im wesentlichen aus einer länglichen Tragschicht aus Kunststoffmaterial und darauf angebrachten Testfeldern bestehen. Es sind jedoch auch Testträger bekannt, die als quadratische oder rechteckige Plättchen gestaltet sind.

Trägergebundene Tests zeichnen sich insbesondere durch die Einfachheit der Handhabung aus. Dies hat dazu geführt, daß derartige Tests in zunehmendem Maße auch für Laien oder für die Verwendung in der Artzpraxis, wo nicht immer spezialisiertes Laborpersonal zur Verfügung steht, angeboten werden.

Soweit bei den bisher bekannten Testträgern ohne Probendosierung gearbeitet wird, soll eine reproduzierbare Tränkung der Reaktionsschicht des Testträgers dadurch erreicht werden, daß die Probe zunächst im Überschuß aufgegeben wird und dieser Überschuß dann abgewischt oder abgewaschen wird. Dadurch wird die Handhabung der Testträger erschwert. Vor allem aber führt das Abwischen oder Abwaschen zu benutzerabhängigen Fehlerquellen, wenn es nicht vollständig ordnungsgemäß durchgeführt wird.

Aus der Deutschen Patentschrift 34 45 816 ist ein streifenförmiger Testträger bekannt, bei dem sich auf einer Tragschicht mehrere saugfähige Testschichten derart nebeneinander angeordnet befinden, daß sie über ihre Kanten miteinander in saugfähigem Kontakt stehen. Dieser bekannte Testträger ist für immunologische Tests gedacht, bei denen außer der Probe ein Elutionsmittel eingesetzt wird. Das Elutionsmittel wird am einen Ende des insgesamt länglichen Testträgers zugeführt und durchwandert die Testschichten in Form eines Flüssigkeitsstroms, der am anderen Ende des Testträgers zum Stillstand kommt. Die Probe wird entweder an der gleichen Stelle wie das Elutionsmittel oder im mittleren Bereich des vorbekannten Testträgers aufgegeben. Das Problem der Dosierung der Probe in einer Reaktionsschicht des Testträgers spielt beim Gegenstand dieser Patentschrift keine Rolle, weil die Probe in präziser Dosierung in den Elutionsmittelstrom eingeführt wird und das Elutionsmittel so lange zugeführt wird, bis es den Testträger vollständig füllt.

Aufgabe der vorliegenden Erfindung ist es, einen Testträger zur Verfügung zu stellen, bei dem eine Reaktionsschicht einerseits reproduzierbar getränkt wird, andererseits aber kein Abwischen oder Abwaschen notwendig ist. Dies soll mit möglichst geringem Aufwand und auch mit kleinen Probenmengen, beispielsweise einem aus einem Stich in die Fingerbeere gewinnbaren Blutstropfen möglich sein.

Die Aufgabe wird durch eine Testträger gemäß den Ansprüchen gelöst.

Die Tragschicht ist bevorzugt langgestreckt wie bei einem üblichen Testtreifen ausgebildet und besteht bevorzugt aus einer Kunststoffolie.

Darauf befinden sich nebeneinander die Aufgabezone, die Nachweiszone und die Saugzone. Die Aufgabezone dient dazu, die Probe dem Testträger zuzuführen. Die Aufgabezone ist mit der Saugzone durch eine kapillaraktive Transportstrecke verbunden.

Der Begriff "kapillaraktive Transportstrecke" ist dahingehend zu verstehen, daß es sich um eine die Aufgabezone und die Saugzone verbindende Strecke handelt, die geeignet ist für einen Flüssigkeitstransport von der Probenaufgabezone zu der Saugzone, wobei der Flüssigkeitstransport auf Kapillarwirkung beruht. Entscheidend für die Kapillarwirkung sind die Oberflächeneigenschaften der beteiligten Materialien und die Abstände der festen Oberflächen in der Transportschicht. Um eine gute Transportwirkung zu erreichen wird ein Material gewählt, welches von der Probenflüssigkeit, gegebenenfalls mit Hilfe geeigneter Netzmittel, gut benetzt wird. Weiterhin ist es für eine gute Kapillarwirkung günstig, wenn die Schicht aus einer Struktur mit dicht beieinander liegenden festen Oberflächen besteht. Konkret kann sie beispielsweise aus einer fasrigen Struktur (z. B. Vlies oder Gewebe) bestehen. Es können jedoch auch andere offene und poröse Strukturen, die einen Flüssigkeitstransport in der Längsrichtung der Schicht ermöglichen, verwendet werden. Besonders bevorzugt enthält die Transportstrecke einen oder mehrere von der Probenaufgabezone zu der Saugzone verlaufende Spalten.

Die Saugzone enthält mindestens eine Saugschicht aus einem saugfähigem Material. Ein saugfähiges Material in diesem Sinne ist jedes Material, das in der Lage ist, Flüssigkeit in sich aufzunehmen. Dies kann insbesondere mit Hilfe der im Zusammenhang mit der Transportstrecke beschriebenen Kapillarwirkung geschehen. Es gibt jedoch auch andere Möglichkeiten, beispielsweise die Wasseraufnahme durch Hydratation, Quellen oder Volumenexpansion der Saugschicht.

Die Nachweiszone ist der Bereich des Testträgers, in dem nach Durchführung der Analyse das nachweisbare Signal gemessen werden kann. Sie enthält eine oder mehrere Reaktionsschichten, in welchen sich Reagenzien befinden, deren Reaktion mit der Probe schließlich zu dem nachweisbaren Signal, im praktisch wichtigsten Fall also zu einem visuell oder photometrisch auswertbaren Farbumschlag führt.

Ein wichtiges Merkmal der Erfindung ist darin zu sehen, daß eine Reaktionsschicht innerhalb der Nachweiszone, parallel zu der Transportstrecke derartig angeordnet ist, daß sie in Flüssigkeitskontakt mit einer in der Transportstrecke transportierten Flüssigkeit steht. Bei der allgemeinsten Form der Erfindung ist es nicht notwendig, daß die Reaktionsschicht der Transportstrecken unmittelbar benachbart ist. Es können auch Zwischenschichten vorhanden sein, welche einen Flüssigkeitstransport von der Transportstrecke zu der Reaktionsschicht erlauben. Die Reaktionsschicht kann zwischen der Tragschicht und der Transportstrecke oder auf der von der Tragschicht abgewandten Seite der Transportstrecke angeordnet sein.

Die Erfindung richtet sich insbesondere auf Tests, bei denen es wichtig ist, daß unabhängig von der Probenmenge die Menge der von der Reaktionsschicht aufgenommenen Flüssigkeit reproduzierbar weitestgehend gleich ist. In dieser Hinsicht ist es besonderes vorteilhaft, wenn die Saugzone, die Transportstrecke und die Reaktionsschicht hinsichtlich ihrer Saugkraft, Sauggeschwindigkeit und Saugvolumen entsprechend aufeinander abgestimmt sind. Ziel dieser Maßnahmen ist es, daß die Flüssigkeit in der Transportschicht mindestens so lange zur Verfügung steht, bis die Reaktionsschicht eine reproduzierbare Flüssigkeitsmenge aufgenommen hat. Danach soll die Flüssigkeitsverbindung zwischen der Transportstrecke und der Reaktionsschicht unterbrochen werden. Näheres wird weiter unten erläutert.

Im folgenden wird die Erfindung anhand der in den Figuren schematisch dargestellten Ausführungsbeispiele näher erläutert. Es zeigen:
- Figur 1: einen erfindungsgemäßen Testträger im Längsschnitt,
- Figur 2: eine andere Ausführungsform eines erfindungsgemäßen Testträgers im Längsschnitt,
- Figur 3: eine Aufsicht auf die Tragschicht eines Testträgers nach Figur 2 in einem noch nicht fertig montierten Zustand,
- Figur 4: ein Querschnitt durch einen Testträger nach Figur 2 entlang der Linie IV-IV,
- Figur 5: eine Funktionskurve für den Zusammenhang zwischen % Remission einer Reaktionsschicht eines erfindungsgemäßen Testträgers und dem Glucosegehalt einer zu untersuchenden Probe.

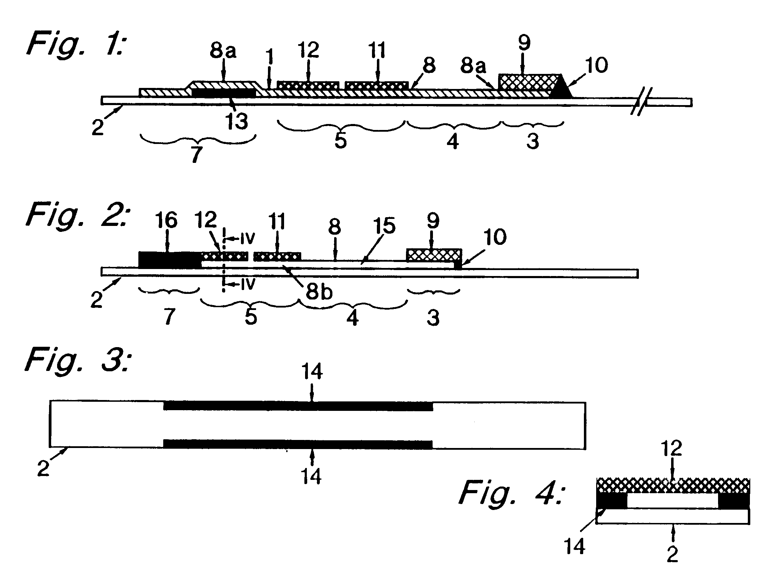

Der in Figur 1 dargestellte Testträger 1 besteht im wesentlichen aus einer Tragschicht 2 und den darauf angeordneten Testschichten. Die Tragschicht 2 ist länglich ausgebildet. Sie ist bevorzugt ungefähr 100 mm lang, ca. 5 - 6 mm breit und 0,3 - 0,4 stark.

Der Testträger läßt sich in seiner Längsrichtung in eine Aufgabezone 3, eine Transportzone 4, eine Nachweiszone 5 und in eine Saugzone 7 unterteilen.

Bei der in Figur 1 dargestellten Ausführungsform wird die kapillaraktive Transportstrecke 8 durch eine sich vom Anfang der Aufgabezone 3 bis ans Ende der Saugzone 7 erstreckende Transportschicht 8 a aus einem faserigen Material (z. B. Vlies oder Gewebe) gebildet. Es genügt, wenn die Transportschicht 8 die Aufgabezone 3 und die Saugzone 7 verbindet. Als besonders geeignetes Material für eine in Form eines Gewebes oder Vlieses ausgebildete Transportschicht hat sich Polyamid erwiesen.

Die Transportschicht 8 a und das in der Aufgabezone darüber befindliche Schutznetz 9 sind gemeinsam mit einem Schmelzkleberstreifen 10 an der Tragschicht 2 befestigt.

Im Bereich der Nachweiszone 5 sind parallel zu der Transportschicht 8 die Reaktionsschichten 11 und 12 parallel und unmittelbar benachbart zu dieser angebracht. Sie können beispielsweise durch auf der Transportschicht 8 a bzw. auf der Tragschicht 2 befestigt sein. Obwohl jeweils zwei Reaktionsschichten dargestellt sind, ist die Erfindung selbstverständlich auch mit nur einer Reaktionsschicht oder mit mehr als zwei Reaktionsschichten ausführbar. Jede der Reaktionsschichten kann auch ihrerseits wiederum aus einem mehrschichtigen Aufbau bestehen, der für die Zwecke dieser Erfindung als Einheit betrachtet wird.

Im Bereich der Saugzone 7 befindet sich parallel zu der Transportschicht zwischen dieser und der Tragschicht 2 eine Saugschicht 13, welche bevorzugt aus einem Material besteht, das aufgrund seiner guten Benetzbarkeit eine hohe Saugkraft hat. Als besonders geeignet hat sich ein Vlies oder Gewebe aus Glasfasern erwiesen.

Bei dem in Figur 2 dargestellten Testträger ist die kapillaraktive Transportstrecke 8 in Form eines kanalförmigen Spaltes 8 b ausgebildet. Der Spalt 8 b wird, wie aus Figur 3 zu erkennen ist, von zwei schmalen Streifen 14 aus Transferklebeband begrenzt, welche der Länge nach auf die Tragschicht 2 aufgeklebt sind. Nach oben hin wird der kanalförmige Spalt durch eine Deckfolie 15 bzw. durch die Reaktionsschichten 11 und 12 abgedeckt. Die Saugzone 7 besteht bei der in den Figuren 2 - 4 dargestellten Ausführungsform im wesentlichen aus einer einzigen blockförmigen Saugschicht 16.

Zur Durchführung einer analytischen Bestimmung wird auf die Aufgabezone 3 eine geringe Probenmenge aufgebracht. Es genügt beispielsweise ein Tropfen Blut von ca. 15 µl Volumen, wie er leicht aus der Fingerbeere gewonnen werden kann. Die Probe gelangt durch das Schutznetz 9 in die Aufgabezone 3 und über die Transportzone 4 aufgrund der Kapillaraktivität der Transportstrecke 8 in die Nachweiszone 5 und weiter bis in die Saugzone 7. Um feststellen zu können, ob eine aufgegebene Blutmenge zur Durchführung der analytischen Bestimmung ausreicht, kann zwischen Nachweiszone 5 und Saugzone 7 auch eine Probenerkennungszone 1 angeordnet sein, die wenn sie mit Probenflüssigkeit in Kontakt kommt, meß- oder erkennbar angibt, daß eine genügende Probenmenge auf den Testträger aufgebracht wurde.

Beim Durchgang durch die Nachweiszone 5 wird ein Flüssigkeitskontakt zu den Reaktionsschichten 11 und 12 hergestellt, so daß sich diese vollsaugen. Wie oben erwähnt, sind die Saugkraft, die Sauggeschwindigkeit und das Saugvolumen der Reaktionsschichten 11, 12, der Transportschicht 8 und der Saugzone 7 (welche wesentlich durch die Eigenschaften der Saugschicht 13, 16 bestimmt wird) so aufeinander abgestimmt, daß die Flüssigkeit in der Nachweiszone 5 mindestens so lange zur Verfügung steht, bis die Reaktionsschichten 11 und 12 eine definierte Menge Flüssigkeit aufgenommen haben, daß sie aber danach durch die Saugzone 7 mindestens so weit wieder abgesaugt wird, daß der Flüssigkeitskontakt zwischen den Reaktionsschichten 11, 12 und der Transportstrecke 8 unterbrochen wird.

Die Geschwindigkeit des Transports der Probenflüssigkeit in der kapillaraktiven Transportstrecke 8 ist einerseits durch die Kapillarkraft und andererseits durch den Strömungswiderstand bestimmt. Je enger die Kapillaren in der Transportstrecke 8 sind, desto höher wird einerseits die Kapillarkraft, desto mehr nimmt aber andererseits der Strömungswiderstand zu. Bei einem Testträger mit einer kanalförmigen Transportstrecke gemäß Figur 2 füllt sich der Kanalspalt beispielsweise bei einer Kanalweite von 0,3 mm in 4 Sekunden, bei einer Spaltweite von 0,2 mm in etwa 6 Sekunden und bei einer Spaltweite von etwa 0,1 mm in 30 Sekunden. Bei einer Spaltweite von 0,05 mm ist die Füllzeit des Kanals größer als 30 Sekunden. Man sieht, daß in diesem Bereich der Einfluß des zunehmenden Strömungswiderstandes gegenüber dem der zunehmenden Kapillarkraft überwiegt und deswegen die Strömungsgeschwindigkeit mit geringer werdender Kapillargröße abnimmt.

Die Sauggeschwindigkeit, mit der sich die Reaktionsschichten 11, 12 füllen, ist von deren Eigenschaften abhängig. Je nach Anwendungsfall werden bei Testträgern sehr verschiedene Reaktionsschichten verwendet. Beispielsweise können die Reagenzien auf einem Papier, Vlies oder auf einer Matrix aus einem porösen Kunststoffmaterial imprägniert sein. Es werden auch Reaktionsschichten mit einem quellfähigen Trägermaterial, beispielsweise einem Gel oder einer Gelatineschicht verwendet. Die Sauggeschwindigkeit, mit der sich die Reaktionsschichten vollsaugen, läßt sich jeweils empirisch bestimmen. Bei der Erfindung soll die Probenflüssigkeit in der kapillaraktiven Transportstrecke 8 mindestens so lange zur Verfügung stehen, bis die Reaktionsschichten 11, 12 im gewünschten Ausmaß die Flüssigkeit aufgesaugt haben. Wenn dies geschieht, ist vor allem von der Geschwindigkeit, mit der die Saugzone die Probenflüssigkeit aufnimmt und von der Probenmenge abhängig. Geht man von einer bestimmten maximalen Probenmenge, die mit dem Testträger untersucht werden kann, aus, so ist es zweckmäßig, daß die gesamte Flüssigkeitsmenge, die die Saugschicht 13, 16 aufnehmen kann, größer ist als die maximale Probenmenge.

Die Saugzone 7 nimmt die Flüssigkeit bevorzugt erheblich langsamer auf, als die Transportstrecke. Dadurch verlangsamt sich der Flüssigkeitsstrom sehr stark, sobald die Flüssigkeitsfront am Beginn der Saugzone 7 angelangt ist. Die weitere Strömung durch die Transportstrecke 8 wird dann durch die Sauggeschwindigkeit der Saugzone bestimmt, d. h. dadurch, wie schnell die Saugzone die Flüssigkeit aufnimmt. Dieser Vorgang soll so langsam sein, daß die Reaktionsschichten 11, 12 ausreichend Zeit haben, sich mit Probenflüssigkeit zu füllen.

Wenn schließlich die auf die Aufgabezone 3 aufgebrachte Probenmenge verbraucht ist, die Saugzone jedoch noch nicht ihr maximales Saugvolumen aufgenommen hat, beginnt die Saugzone 7 die kapillaraktive Transportstrecke 8 leerzusaugen. Dazu ist erforderlich, daß die Saugkraft der Saugzone 7 größer ist als die der kapillaraktiven Transportstrecke 8. Dies kann beispielsweise dadurch erreicht werden, daß die in der Saugzone 7 enthaltene Saugschicht 13, 16 aus einem kapillaraktiven Material mit besonders hoher Benetzbarkeit für die Probenflüssigkeit, beispielsweise aus Glasfasern, besteht.

Beim Entleeren der kapillaraktiven Transportstrecke 8 soll der Flüssigkeitskontakt zu den Reaktionsschichten 11, 12 unterbrochen werden, ohne daß diese in nennenswertem, (d. h. die Genauigkeit beeinträchtigendem Umfang) an Flüssigkeit verlieren. Deswegen wird es bevorzugt, daß auch die Reaktionsschichten 11, 12 eine höhere Saugkraft als die kapillaraktive Transportstrecke 8 haben.

Die zusätzliche Transportzone 4 zwischen Aufgabezone 3 und Nachweiszone 5 kann zweckmäßig sein, wenn ein Testträger gewünscht wird, bei dem die Aufgabezone 3 und die Nachweiszone 5 nicht sehr dicht beieinander liegen.

Das folgende Beispiel dient der Erläuterung der Erfindung.

### Beispiel 1

### Testträger zur Messung von Glucose

Der Testträger wird gemäß Figuren 2, 3 und 4 aufgebaut:
Die Tragschicht 2 besteht aus einer Polyesterfolie (Dicke 0,5 mm, Länge 77 mm, Breite 6 mm). Hierauf sind mit zwei parallel angeordneten, seitenbündig abschließenden Transferklebebändern 14 (doppelseitig klebendes Klebeband) (Dicke 0,1 mm, Länge 43 mm, Breite 1,5 mm), das Schutznetz 9, eine Deckfolie 15 und die Reaktionsschichten 11 und 12 befestigt.

Das Schutznetz 9 ist ein Polyesternetz (PE 280 HC, Schweizer Seidengazefabrik Thal, Schweiz), Maschenweite 280 µm, Dicke 0,2 mm, Länge 8 mm, Breite 6 mm. Es ist an einer Seite mit einem Schmelzklebestreifen 10 auf der Polyesterfolie (2) befestigt. Die Deckfolie 15 ist eine einseitig mit Agarose beschichtete Polyesterfolie (Gel-Fix, Serva, Heidelberg, Bundesrepublik Deutschland) (Dicke 0,18 mm, Länge 10 mm, Breite 6 mm), deren beschichtete Seite zur Tragschicht 2 gerichtet ist.

Die Reaktionsschichten 11 und 12 werden folgendermaßen hergestellt:
198 g Acrylsäureester-Copolymer-Dispersion (Acronal 14D der BASF, Ludwigshafen, Bundesrepublik Deutschland, 55 %ig in Wasser)
174 g gequollene, hochviskose Methylhydroxyethylcellulose (0,5 %ig in Wasser)
336 g Kieselgur
336 g Titandioxid
0,95 g Tetraethylammonium-perfluoroctansulfonat
40 g 0,5 M Phosphatpuffer, pH 5,5
23 g Methanol
46 g 1-Hexanol
69 g Aceton
65 g Wasser
werden zu einer homogenen ersten Beschichtungsmasse verarbeitet und mit 0,18 mm Spalthöhe auf ein 0,20 mm dickes Polyester-Filtergewebe (2 F 777, Schweizer Seidengazefabrik Thal, Schweiz) beschichtet und getrocknet.

Auf den so erhaltenen, beschichteten Träger wird eine zweite Beschichtungsmasse bestehend aus
102g Acrylsäureester-Copolymer-Dispersion (Acronal 14D der BASF, Ludwigshafen, Bundesrepublik Deutschland, 55 %ig in Wasser)
38 g gequollene, hochviskose Methylhydroxyethylcellulose (0,5 %ig in Wasser)
3 g Natriumdodecylbenzolsulfonat
36 KU Glucoseoxidase
1050 KU Peroxidase
1,48 g 3,3ʹ, 5,5ʹ-Tetramethylbenzidin
0,53 g 1-Phenylsemicarbazid
28 g 1-Methoxy-2-propanol
40 g 1-Hexanol
38 g Wasser
die zu einer homogenen Masse verarbeitet wurden, mit 0,02 mm Spalthöhe aufgetragen und getrocknet.

Die zwei verwendeten Reaktionsschichten 11 und 12 sind je 6 mm lang und 6 mm breit. Sie sind so nebeneinander befestigt, daß kein Spalt zwischen ihnen bleibt.

Die Saugschicht 16 besteht aus einem Glasfaservlies eines Flächengewichts von 60 mg/m² (Dicke 0,3 mm, Länge 12 mm, Breite 6 mm).

Zur Messung von Glucose werden 20 µl Blut auf die Aufgabezone 3 des Testträgers gegeben. Nach zwei Minuten bei Raumptemperatur werden die Reaktionsschichten 11 und 12 bei einer Wellenlänge von 660 nm reflexionsphotometrisch vermessen.

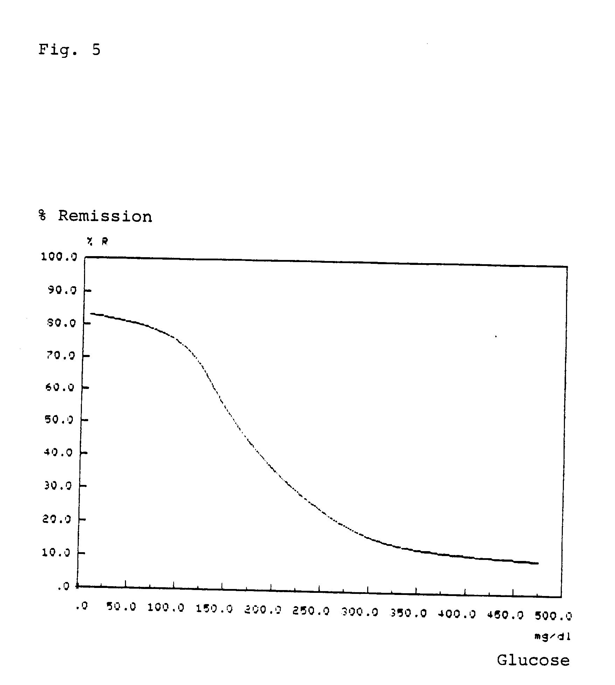

Mit Proben bekannter Glucosekonzentration wurde eine Funktionskurve (Abb. 5) erstellt, in der ein Meßwert in % Remission einer bestimmten Glucosekonzentration zugeordnet wurde. Mit Hilfe einer solchen Kurve können auch Proben mit unbekanntem Glucosegehalt quantitativ vermessen werden.

## Patentansprüche

1. Testträger (1) für die analytische Bestimmung von Bestandteilen von Körperflüssigkeiten aus einer bestimmten maximalen Probenmenge mit einer Tragschicht (2) und mehreren darauf angeordneten Testschichten, die wenigstens teilweise in Flüssigkeitsaustausch ermöglichendem Kontakt zueinander stehen,
wobei
eine Aufgabezone (3) für die Aufgabe einer Probe der Körperflüssigkeit,
eine Nachweiszone (5) für die Erzeugung eines nachweisbaren für die analytische Bestimmung charakteristischen Signals, welche eine Reaktionsschicht (11, 12) enthält und
eine Saugzone (7) mit einer Saugschicht (13, 16) aus einem saugfähigem Material,
in dieser Reihenfolge nebeneinander auf der Tragschicht (2) angeordnet sind,
dadurch gekennzeichnet, daß
zwischen der Aufgabezone (3) und der Saugzone (7) eine kapillaraktive Transportstrecke (8) derartig angeordnet ist, daß sie die Aufgabezone (3) und die Saugzone (7) verbindet und
die Reaktionsschicht (11, 12) parallel zu der Transportstrecke (8) zwischen der Aufgabezone (3) und der Saugzone (7) derartig angeordnet ist, daß sie in Flüssigkeitskontakt mit einer in der Transportstrecke (8) transportierten Flüssigkeit steht.

2. Testträger nach Anspruch 1, dadurch gekennzeichnet, daß das gesamte Saugvolumen der Saugschicht (13, 16) größer als die maximale Probenmenge ist.

3. Testträger nach Anspruch 1, dadurch gekennzeichnet, daß die Sauggeschwindigkeit der Reaktionschicht (11, 12) größer ist als die Sauggeschwindigkeit der Saugzone (7).

4. Testträger nach Anspruch 1, dadurch gekennzeichnet, daß die Saugkraft der Saugzone (7) größer als die der kapillaraktiven Transportstrecke (8) ist.

5. Testträger nach Anspruch 1, dadurch gekennzeichnet, daß die Saugkraft der Reaktionsschicht (11, 12) größer als die der kapillarakti- ven Transportstrecke (8) ist.

6. Testträger nach Anspruch 1, dadurch gekennzeichnet, daß zwischen der Aufgabezone (3) und der Nachweiszone (5) eine Flüssigkeitstransportzone (4) vorgesehen ist.

7. Testträger nach Anspruch 1, dadurch gekennzeichnet, daß die kapillaraktive Transportstrecke (8) eine Transportschicht (8a) aus einem fasrigen Material einschließt.

8. Testträger nach Anspruch 1, dadurch gekennzeichnet, daß die kapillaraktive Transportstrecke (8) einen Spalt (8b) von mindestens 0,05mm Weite bevorzugt etwa 0,15 - 0,20 mm Weite einschließt.

## Claims

1. Test carrier (1) for the analytical determination of components of body fluids from a precise maximum amount of sample with a carrier layer (2) and several test layers arranged thereupon which at least partly are in contact with one another making possible liquid exchange, whereby an application zone (3) for the application of a sample of the body fluid, a detection zone (5) for the production of a detectable signal characteristic for the analytical determination, which contains a reaction layer (11, 12) and an absorption zone (7) with an absorption layer (13, 16) of an absorptive material are arranged next to one another in this sequence on the carrier layer (2), characterised in that, between the application zone (3) and the absorption zone (7), a capillary-active transport path (8) is arranged in such a manner that it connects the application zone (3) and the absorption zone (7) and the reaction layer (11, 12) is arranged parallel to the transport path (8) between the application zone (3) and the absorption zone (7) in such a manner that it is in liquid contact with a liquid transported in the transport path (8).

2. Test carrier according to claim 1, characterised in that the total absorption volume of the absorption layer (13, 16) is greater than the maximum amount of sample.

3. Test carrier according to claim 1, characterised in that the speed of absorption of the reaction layer (11, 12) is greater than the speed of absorption of the absorption zone (7).

4. Test carrier according to claim 1, characterised in that the absorptive power of the absorption zone (7) is greater than that of the capillary-active transport path (8).

5. Test carrier according to claim 1, characterised in that the absorptive power of the reaction layer (11, 12) is greater than that of the capillary-active transport path (8).

6. Test carrier according to claim 1, characterised in that a liquid transport zone (4) is provided between the application zone (3) and the detection zone (5).

7. Test carrier according to claim 1, characterised in that the capillary-active transport path (8) includes a transport layer (8a) of a fibrous material.

8. Test carrier according to claim 1, characterised in that the capillary-active transport path (8) includes a slot (8b) of at least 0.05 mm width, preferably about 0.15 - 0.20 mm width.

## Revendications

1. Support de test (1) pour la détermination analytique de constituants de fluides corporels à partir d'une quantité maximale déterminée d'échantillon, comportant une couche de support (2) et plusieurs couches de test, disposées sur celle-ci, qui sont entre elles en un contact permettant un échange au moins partiel de liquides, dans lequel sont disposées, de façon adjacente entre elles, cet ordre, sur la couche de support (2) :
une zone d'application (3) destinée à la réception d'un échantillon du fluide corporel,
une zone de détection (5), destinée à l'obtention d'un signal détectable caractéristique pour la détermination analytique, qui contient une couche de réaction (11, 12), et
une zone d'absorption (7) comportant une couche absorbante (13, 16) en un matériau absorbant,
caractérisé en ce que, entre la zone d'application (3) et la zone d'absorption (7), une conduite de transport capillaire (8) est disposée de façon à relier entre elles la zone d'application (3) et la zone d'absorption (7), et que la couche de réaction (11, 12) est disposée parallèlement à la conduite de transport (8) entre la zone d'application (3) et la zone d'absorption (7) de telle façon qu'elle soit en contact liquide avec un fluide transporté par la conduite de transport (8).

2. Support de test selon la revendication 1, caractérisé en ce que le volume absorbant total de la couche absorbante (13, 16) est supérieur à la quantité maximale d'échantillon.

3. Support de test selon la revendication 1, caractérisé en ce que la vitesse d'absorption de la couche de réaction (11, 12) est supérieure à la vitesse d'absortion de la zone d'absorption (7).

4. Support de test selon la revendication 1, caractérise en ce que la force d'absorption de la zone d'absorption (7) est supérieure à celle de la conduite de transport capillaire (8).

5. Support de test selon la revendication 1, caractérisé en ce que la force d'absorption de la couche de réaction (11, 12) est supérieure à celle de la conduite de transport capillaire (8).

6. Support de test selon la revendication 1, caractérisé en ce qu'une zone de transport de fluide (4) est prévue entre la zone d'application (3) et la zone de détection (5).

7. Support de test selon la revendication 1, caractérisé en ce que la conduite de transport capillaire (8) comprend une couche de transport (8a) faite d'une matière fibreuse.

8. Support de test selon la revendication 1, caractérisé en ce que la conduite de transport capillaire (8) comprend une fente (8b) ayant une largeur d'au moins 0,05 mm, de préférence environ 0,15-0,20 mm.
